(19) Europäisches Patentamt
European Patent Office
Office européen des brevets

(11) **EP 0 973 445 B1**

(12) **EUROPÄISCHE PATENTSCHRIFT**

(45) Veröffentlichungstag und Bekanntmachung des Hinweises auf die Patenterteilung:
**07.04.2004 Patentblatt 2004/15**

(21) Anmeldenummer: **98914812.7**

(22) Anmeldetag: **24.02.1998**

(51) Int Cl.7: **A61B 5/103**

(86) Internationale Anmeldenummer:
**PCT/DE1998/000540**

(87) Internationale Veröffentlichungsnummer:
**WO 1998/037809 (03.09.1998 Gazette 1998/35)**

(54) **LAHMHEITSDIAGNOSE**

LAMENESS DIAGNOSIS

DIAGNOSTIC DE LA BOITERIE

(84) Benannte Vertragsstaaten:
**AT BE CH DE ES FR GB IE IT LI NL SE**

(30) Priorität: **25.02.1997 DE 19707413**

(43) Veröffentlichungstag der Anmeldung:
**26.01.2000 Patentblatt 2000/04**

(73) Patentinhaber: **marquis Tiermedizintechnik**
**89542 Herbrechtingen (DE)**

(72) Erfinder: **Falaturi, Parvis**
**34305 Niedenstein (DE)**

(74) Vertreter: **Lorenz, Werner, Dr.-Ing. et al**
**Lorenz & Kollegen**
**Patent- und Rechtsanwaltskanzlei**
**Alte Ulmer Strasse 2-4**
**89522 Heidenheim (DE)**

(56) Entgegenhaltungen:
**GB-A- 2 278 198 US-A- 4 233 845**
**US-A- 4 774 679**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die Erfindung betrifft eine Vorrichtung nach dem Oberbegriff von Anspruch 1.

Bekannte Verfahren und Vorrichtung zur Erzeugung von Signalen, mittels derer beurteilt werden kann, ob ein Reittier lahmt, arbeiten z.B. mit am Erdboden befindlichen Meßgegenständen wie Kraftmeßplatten oder mit Luft gefüllten Vierkantschläuchen, die häufig im Boden einer Vorführbahn eingelassen sind, damit das Reittier natürlich und ungehemmt darüber hinwegläuft. Die von den Meßgegenständen abgegebenen Signalen werden gesammelt, aufgezeichnet und ausgewertet. Da das Reittier auf einer Vorführbahn laufen muß, wird es häufig in seiner natürlichen Bewegung gehemmt, was die Messungen verfälschen kann.

Ein weiteres Verfahren ist die Kinematographie, bei der bestimmte Stellen der Extremitäten des Reittiers während seiner Bewegung mit einer Hochfrequenzkamera gefilmt werden. Nachteile dieser Technik bestehen vor allem darin, daß die Entwicklung des Filmes Zeit benötigt, wodurch die Bewertung verzögert wird, und für eine automatische Auswertung des Films sehr aufwendig ist.

Weiterhin sind Verfahren und Vorrichtungen bekannt, die an den Gelenken des Reittiers angebrachte Sensoren aufweisen, die die Gelenkwinkel bei der Bewegung des Reittiers messen. Ein Nachteil dieser als Elektrogoniometrie bezeichneten Methode ist es, daß Messungen aufgrund der aufwendigen Erfassung und komplizierten Auswertung der Meßwerte meist nur in Tierkliniken vorgenommen werden können.

Weiterhin sind Verfahren und Vorrichtungen der eingangs bezeichneten Gattungen bekannt (US-PS 4 774 679), die mit am Bein eines Reittiers angebrachten Beschleunigungssensoren arbeiten und insbesondere einer genauen Analyse der Schrittlänge dienen sollen. Dazu werden diejenigen Teile der Sensorsignale zur Auswertung herangezogen, die oberhalb eines vorgewählten Schwellwertes liegen. In der Praxis hat sich jedoch gezeigt, daß eine derartige Auswertung nicht zu eindeutigen Ergebnissen und insbesondere nicht zu einer exakten Beantwortung der Frage führen kann, ob ein Reittier lahm geht oder nicht. Erst recht kann eine nur geringgradige Lahmheit auf diese Weise nicht ermittelt werden.

Schließlich ist es bekannt (GB-A-2 278 198), mit Hilfe eines Beschleunigungsmessers, der an irgendeiner Extremität eines Lebewesens, insbesondere eines Pferdes befestigt ist, auf den Zustand des Bodens zu schließen, auf dem sich das Lebewesen gerade bewegt. Zu diesem Zweck werden die ermittelten Signale mit einer Vielzahl von Signalen verglichen, die von Auffußvorgängen auf unterschiedlichen Böden bzw. Geläufen stammen und in einer Bibliothek abgelegt sind. Außerdem soll durch einen Vergleich der Signale, die von einem Pferd, das an einem Bein verletzt ist, erhalten werden, mit den von einem gesunden Pferd erhaltenen Signalen auf den momentanen Zustand des verletzten Pferdes geschlossen werden.

Aufgabe der Erfindung ist es daher die Vorrichtungen der eingangs bezeichneten Gattungen dahingehend zu verbessern, daß sie bei relativ geringem Meßund Auswerteaufwand zur Diagnose der Lahmheit eines Reittiers geeignet sind, ohne daß dabei das Pferd auf einer besonders präparierten Vorführbahn laufen oder in eine Tierklinik gebracht werden muß.

Diese Aufgabe wird durch eine Vorrichtung nach Anspruch 1 gelöst. Weitere vorteilhafte Merkmale der Erfindung ergeben sich aus den jeweiligen Unteransprüchen.

Im folgenden wird die Erfindung anhand eines bevorzugten Ausführungsbeispiels mit begleitenden Zeichnungen erläutert. Darin ist:

Fig. 1 die Seitenansicht eines Reittiers mit an seinen Extremitäten befestigten Einrichtungen zur Meßwerterfassung und -übertragung,

Fig. 2 eine Unteransicht eines Hufs des Reittiers mit einem daran angebrachten Drucksensor;

Fig. 3 ein schematisches Blockschaltbild eines bevorzugten Ausfuhrungsbeispiels der erfindungsgemäßen Vorrichtung zur Meßwerterfassung und -übertragung,

Fig. 4 ein schematisches Blockschaltbild eines bevorzugten Ausführungsbeispiels der erfindungsgemäßen Vorrichtung zur Weiterverarbeitung der Meßsignale,

Fig. 5 ein an einem Zeitstrahl schematisch dargestellter zeitlicher Ablauf einer typischen Schrittfolge eines Pferds in der Gangart Schritt,

Fig. 6 ein Detailausschnitt aus einem digitalisierten und geglätteten Meßsignal,

Fig. 7 einen grob schematischen Programmlaufplan für eine Lahmheitsdiagnose bei Anwendung der Vorrichtung nach Fig. 3 und 4,

Fig. 8 ein Meßsignal bei Anwendung eines an einem Huf eines Reittiers angebrachten Beschleunigungssensors, und

Fig. 9 ein Meßsignal bei Anwendung eines an einem Röhrbein eines Reittiers angebrachten Beschleunigungssensors.

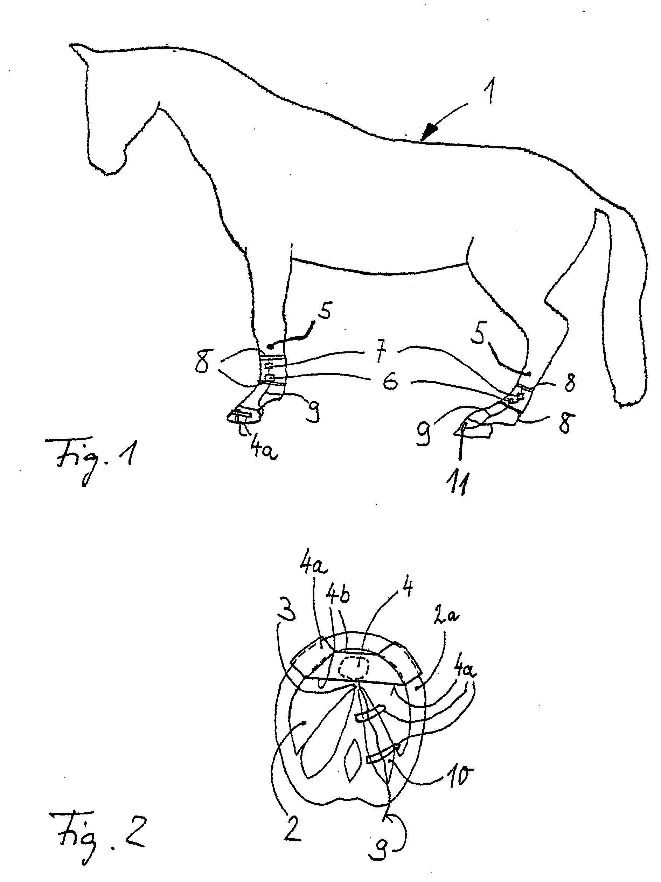

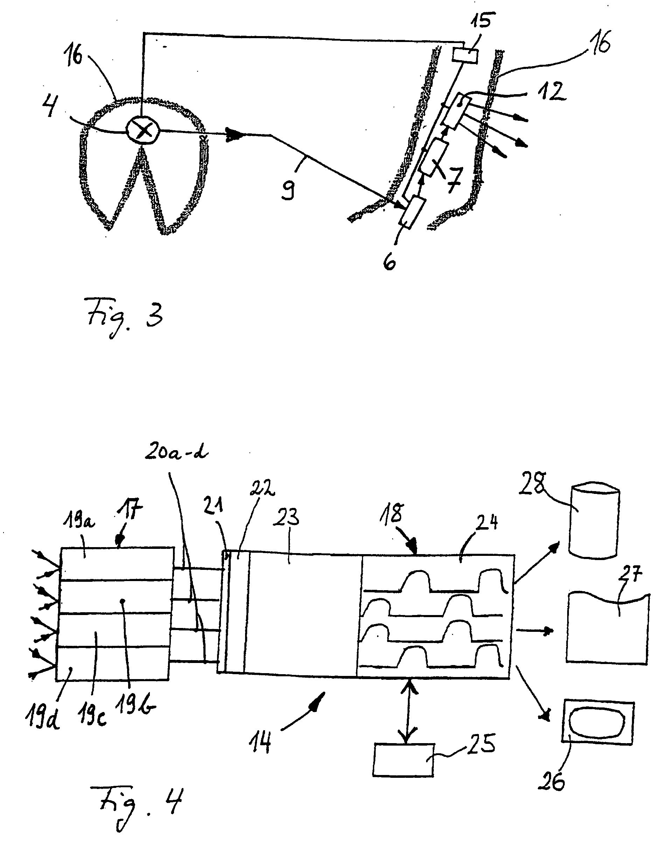

Aus Fig. 1 und 2 wird ersichtlich, daß an den Extremitäten eines Reittiers 1, hier eines Pferds, bestimmte Teile der erfindungsgemäßen Vorrichtung zur Signalerzeugung und Signalaufbereitung in einer Weise angebracht sind, daß das Reittier 1 in seiner Bewegungsfreiheit nicht eingeschränkt ist. Wie insbesondere Fig. 2 schematisch zeigt, weist ein Huf eines Pferdes an seiner Unterseite eine Hufmulde 2, die von einer Hufwand 2a begrenzt ist, und eine Strahlspitze 3 auf. Ein Drucksensor 4, der vorzugsweise aus einem Drucksensor der Typenreihe FSR der Fa. Interlink besteht, ist kurz vor der Strahlspitze 3 mit einem Klebeband 4a befestigt. Geeignet hierfür ist insbesondere das Klebeband "Tesa 5441", das beim Anlegen von medizinischen Verbänden verwendet wird. Das Klebeband 4a verläuft einmal quer über den Sensor 4, erstreckt sich nach beiden Seiten weiter in der Hufmulde 2 bis zur Hufwand 2a, die es vollständig umgreift, und endet jeweils auf der Außenseite des Hufs, wo beide freie Enden des Klebebands 4a mit einem zweiten Stück desselben Klebebands 4a noch zusätzlich überklebt werden. Aufgrund seiner durch die Muldenlage geschützten Position läßt sich der Sensor 4 so anbringen, daß er vom Reittier 1 durch dessen Bewegung oder durch Scharren mit dem Huf weder beschädigt noch abgerissen werden kann. Um zu vermeiden, daß der Sensor 4 ein Dauersignal abgibt, das nicht von einem Auftreten bzw. Auffussen herrührt, sondern von Schmutzpartikeln verursacht wird, die über Ränder 4b des Klebebands 4a eindringen und sich zwischen Sensor 4 und Hufmulde 2 oder zwischen Sensor 4 und Klebeband 4a verkeilen, kann auch die gesamte Hufmulde 2 mit Klebeband 4a abgedeckt werden.

Wie Fig. 1 und 2 weiter zeigen, sind am Röhrbein 5 einer vorderen Extremität des Reittiers 1 ein Meßverstärker 6 und eine an ihn angeschlossene Funktionseinheit 7 mit Hilfe von verstellbaren Lederriemen 8 angebracht. Der Meßverstärker 6, die Funktionseinheit 7 und der Sensor 4 sind durch schematisch dargestellte Kabel 9 miteinander verbunden, die sowohl der Spannungsversorgung als auch der Signalübertragung dienende Leitungen enthalten und ebenfalls mit Stücken des Klebebands 4a am Huf und/oder Röhrbein 5 befestigt sein können. Dabei kann das Kabel 9 im Bereich des Hufs durch eine der beiden äußeren Strahlfurchen 10 (Fig. 2) der Hufmulde 2 vom Sensor 4 zur Außenseite des Hufes und von dort an der Außenseite des Hufes entlang weiter nach oben zum Meßverstärker 6 geführt sein, der am Röhrbein 5 befestigt ist. Die vom Sensor 4 kommenden Signale werden dort verstärkt, in der Funktionseinheit 7 vorzugsweise digitalisiert und dann weiterverarbeitet.

In Fig. 1 ist auch eine hintere Extremität mit einem beschlagenen Huf dargestellt. Bei einem dort gezeigten Sensor 11 handelt es sich um einen Dehnungsmeßstreifen, der etwas seitlich in der Nähe der Hufvorderseite angebracht ist, die Bewegung, insbesondere die Kompression der Hufwand 2a registriert und elektrische Signale analog zu Fig. 1 und 2 erzeugt sowie zur Signalaufbereitung und -übertragung weitergibt. Bevorzugt werden hierfür Dehnungsmeßstreifen verwendet, die unter der Bezeichnung LL 11-3/350 von der Fa. Hottinger Baldwin Meßtechnik, Darmstadt, vertrieben und zweckmäßig mit dem von derselben Firma gelieferten Schnellklebemittel X60 am Huf befestigt werden.

Es wurden hier verschiedene Sensoren 4, 11 dargestellt, um zu zeigen, daß unterschiedliche Sensoren zur Erfassung von Daten von Hufen des Reittiers 1 geeignet und dort auch anbringbar sind. Für die folgende Beschreibung soll jedoch angenommen werden, daß zum Zwecke einer guten Vergleichbarkeit der Signale miteinander an allen vier Extremitäten des Reittiers 1 gleiche Sensoren an gleichen Stellen am jeweiligen Huf angeordnet sind.

Fig. 3 zeigt in einem schematischen Blockschaltbild die Signalerfassung an einer Extremität durch den Sensor 4 mit nachgeschaltetem Meßverstärker, die Sigrialaufbereitung durch die nachgeschaltete, im Ausführungsbeispiel aus einem A/D-Wandler bestehende Funktionseinheit 7 und die bevorzugte drahtlose Signalübertragung mittels eines der Funktionseinheit 7 nachgeschalteten, in Fig. 1 und 2 nicht dargestellten Senders 12 an eine nicht am Reittier 1 befindliche, vorzugsweise transportable Station 14. Diese Signalübertragung kann in an sich bekannter Weise erfolgen (z.B. US-PS 4 774 679). Wesentlich dabei ist jedoch, daß der Sender 12 auch am Röhrbein des Reittiers 1 und nicht am Sattel od. dgl. oder gar am Reiter befestigt wird. Außerdem ist in Fig. 3 eine zweckmäßig ebenfalls am Röhrbein 5 befestigte Spannungsversorgung 15 in Form einer Batterie oder dgl. dargestellt, die mit dem Sensor 4, dem Meßverstärker 6, der Funktionseinheit 7 und dem Sender 12 verbunden ist. In Fig. 3 schematisch eingezeichnete Konturen 16 sollen dabei den Ort der Anbringung der jeweiligen Schaltelemente am Huf bzw. Röhrbein 5 zeigen. Für jede Extremität des Reittiers 1 ist eine in Fig. 3 gezeigte Anordnung vorgesehen und auf die in den Fig. 1 und 2 dargestellte Weise an der Extremität befestigt, wobei die Signale aller Sensoren 4 jeweils einem zugeordneten Sender 12 zugeführt werden. Alternativ kann vorgesehen sein, die Einrichtungen 6, 7, 12 und 15 in eine Manschette oder Gamasche für das Reittier 1 zu integrieren.

In Fig. 4 sind Einzelheiten der räumlich vom Reittier 1 getrennten Station 14 vorgesehen. Sie besteht aus einer Empfängereinheit 17 und einer Auswerteeinheit 18. Die Empfängereinheit 17 empfängt die von den vier Sendern 12 auf verschiedenen Trägerfrequenzen gesendeten digitalen Signale jeweils separat auf einem Kanal 19a bis 19d.

Die Empfängereinheit 17 gibt ihre Signale an vier Ausgängen 20a bis 20d kanalweise getrennt an die Auswerteeinheit 18 weiter, an deren Eingang 21 mit Hilfe einer Zeitmeß- und Synchronisiereinrichtung 22 dafür gesorgt ist, daß die ankommenden digitalen Meßsignale in der genauen Abfolge ihres Empfangs relativ zu einem für alle Kanäle gemeinsamen zeitlichen Bezugspunkt als Daten in einem Zwischenspeicher 23 der Auswerteeinheit 18 geordnet zwischengespeichert werden können. Pro Zeiteinheit werden dabei für jeden Kanal gleich viele Meßwerte zwischengespeichert.

Die Zwischenspeicherung aller Daten erfolgt beispielsweise dadurch. daß die Ausgänge 20a bis 20d mittels der Zeitmeß- und Synchronisiereinrichtung 22 mit einer Frequenz zyklisch wiederholt abgefragt werden, die dem Vierfachen der Meßfrequenz entspricht. Beträgt die Meßfrequenz in allen Kanälen einheitlich 200 Hz, erfolgt die Abfrage z.B. mit 800 Hz. Wird dabei der erste abgefragte Wert zur Definition eines gemeinsamen zeitlichen Nullpunktes der gesamten Messung verwendet, besitzt der zweite abgefragte Wert die Zeitinformation 1,25 msec, der dritte Wert die Zeitinformation 2,5 msec usw. und der fünfte Wert die Zeitinformation 5 msec, jeweils bezogen auf den gemeinsamen zeitlichen Nullpunkt. Dadurch werden alle in den verschiedenen Kanälen gesendeten Meßwerte auch tatsächlich gelesen und gespeichert.

Die zu den Zeitinformationen gehörenden Meßwerte stellen Amplitudenwerte f (t) des Meßsignals in digitaler Form dar. Die zugehörige Zeitinformation ergibt sich z.B. durch den Platz des Meßwerts im Zwischenspeicher 23, indem z.B. der 1., 5., 9. usw. Meßwert im Zwischenspeicher 23 vom Ausgang 20a bzw. aus dem Kanal 19a, der 2., 6., 10. usw. Meßwert vom Ausgang 20b bzw. aus dem Kanal 19b usw. stammt. Da außerdem jeder der vier Sensoren 4 einem der vier Kanäle fest zugeordnet ist und die Zeitmeß- und Synchronisiereinrichtung 22 z.B. immer periodisch mit dem Ausgang 20a beginnt und mit dem Ausgang 20d endet, kann jeder in den Zwischenspeicher 23 geschriebene Meßwert auch einem der vier Hufe bzw. Beine eindeutig zugeordnet werden. Im Ausführungsbeispiel ist die Zuordnung beispielsweise derart, daß der linke Hinterhuf dem Kanal 19a, der linke Vorderhuf dem Kanal 19b, der rechte Hinterhuf dem Kanal 19c und der rechte Vorderhuf dem Kanal 19d zugeordnet ist.

Von dem Zwischenspeicher 23 aus werden die Werte von einer ebenfalls in der Auswerteeinheit 18 befindlichen Auswertelogik 24 gelesen, bei Bedarf unter Anwendung eines geeigneten Tiefpaßfilters, der beispielsweise Frequenzen unterhalb von 10 bis 20 Hz durchläßt, geglättet und miteinander in Beziehung gesetzt.

Nach der Glättung der Signale aus jeweils einem Kanal sollten sie in bevorzugter Weise für ihre weitere Auswertung etwa so glatt sein, wie es im rechten Teil von Fig. 4 schematisch dargestellt ist.

Die Auswerteeinheit 18 ist vorzugsweise durch einen Mikrorechner realisiert. Sie verfügt weiter über einen mit der Auswertelogik 24 zusammenwirkenden Arbeitsspeicher 25 und kann verschiedene Ausgabegeräte wie ein Display 26, ein Druckwerk 27 und ein Diskettenlaufwerk 28 für eine Ausgabe der Ergebnisse ihrer Auswertungen ansteuern. Die Ausgabegeräte 26 bis 28 können vor allem dazu benutzt werden, die von den Sensoren 4, 11 erhaltenen elektrischen Signale auf einem Bildschirm sichtbar zu machen oder komplett anzudrucken.

Die Auswertelogik 24 ist durch ihre Programmierung dazu eingerichtet, in Ergänzung zum oben erwähnten Lesen und Glätten der Meßdaten wenigstens gewisse Berechnungen anhand der in den Signalen enthaltenen Daten durchzuführen, um dadurch für die Lahmheitsdiagnose geeignete Kenngrößen zu liefern, wie im folgenden im Zusammenhang mit zwei verschiedenen, beispielhaften Messungen und ihren Auswertungen beschrieben wird.

An allen vier Hufen werden gemäß Fig. 2 gleiche, vorzugsweise im Handel allgemein erhältliche Drucksensoren 4 und die weiteren Bauteile zur Signalaufbereitung und - übertragung wie oben beschrieben am Reittier 1 befestigt. Des weiteren wird die Station 14 in den Sendebereich der Sender 12 gebracht, die Signalerfassung aktiviert und das Reittier 1 mit oder ohne Reiter dazu gebracht, auf einem ihm bekannten, idealerweise ebenen Gelände in gerader Richtung im Schritt zu gehen und dabei nicht in Trab oder Galopp zu fallen. Eine geringe, gleichmäßige Steigung dürfte vorhanden sein, solange kein besonderer Wert auf einen Vergleich der Meßwerte vom vorderen Extremitätenpaar mit denen des hinteren Extremitätenpaars gelegt wird und sich die Auswertung infolgedessen auf rechts/links-Vergleiche am gleichen Extremitätenpaar beschränkt. Die Bewegung sollte in bevorzugter Weise wenigstens 30 bis 40 vollständige, aufeinanderfolgende Schrittfolgen der Gangart Schritt enthalten, während derer die von den Sensoren 4 erfaßten Meßdaten vorzugsweise kontinuierlich an die Station-14 gesendet werden. Die Station 14 speichert alle für die Auswertung relevanten Daten. Sind die Schrittfolgen des Reittiers 1 vollständig durchgerührt, kann die Messung durch Deaktivierung der Sensoren 4 oder der Übertragung beendet und können die Sensoren 4 vom Reittier 1 entfernt werden.

Im folgenden wird davon ausgegangen, daß die so erfaßte Bewegung des Reittiers 1 aus vierzig vollständigen, ununterbrochenen Schrittfolgen oder Schrittzyklen der Gangart Schritt mit der Abfolge

links hinten - links vorn - rechts hinten - rechts vorn - links hinten... usw.

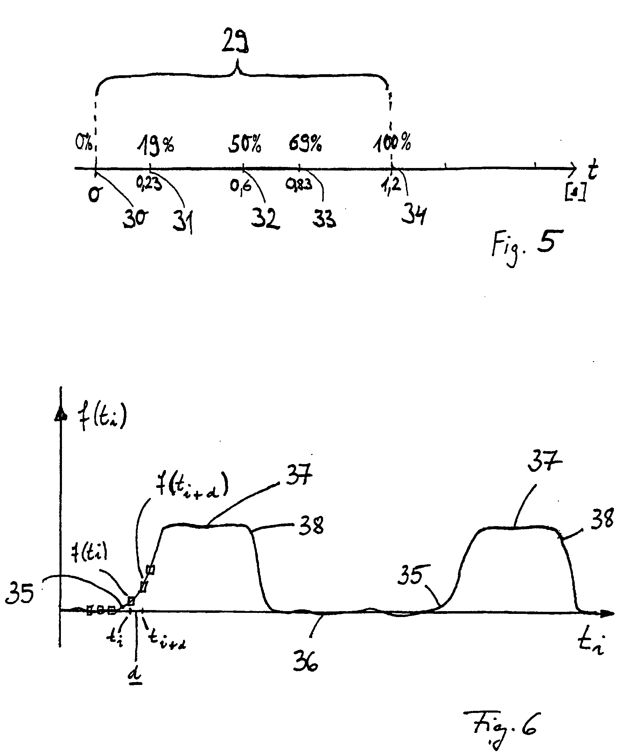

besteht. Je nach Bewegungstempo kann eine einzige solche Schrittfolge, beginnend mit dem Auftreten des linken hinteren Hufs am Erdboden und endend mit dessen erneutem Auftreten, beispielsweise 1,2 Sekunden (zwölf Zehntelsekunden) dauern. Diese Zeitspanne wird im folgenden Zyklusdauer 29 (Fig. 5) genannt. Sie kann ebenso unter Verwendung eines anderen Hufs gebildet werden.

In Fig. 5 ist eine solche Schrittfolge mit einem zugehörigen Zeitstrahl dargestellt. Dabei können die Auftreffzeitpunkte der einzelnen Hufe auch durch die jeweils bis dort erfolgten Teile der betreffenden Zyklusdauer 29 in Prozent ausgedrückt anstatt in einer Zeiteinheit wie "Sekunden seit Zyklusbeginn" gemessen werden. Wenn 100% der Länge einer kompletten Zyklusdauer 29 entsprechen, markiert das Aufsetzen des linken, hinteren Hufs am Erdboden bei 30 den Beginn des Zyklus entsprechend 0% der Zyklusdauer 29. Nach ca. 0,23 sec oder an einer Stelle 31, die z.B. nach ca. 19% der Zyklusdauer 29 erreicht ist, setzt der vordere linke Huf auf. Der rechte hintere Huf setzt dann z:B. nach 0,6 sec bzw. an einer Stelle 32 entsprechend einem Ablauf von 50% der Zyklusdauer 29 auf dem Erdboden auf. An einer Stelle 33, die z.B. nach ca. 0,83 sec bzw. nach Ablauf von ca. 69% der Zyklusdauer 29 erreicht ist, setzt dann der rechte vordere Huf auf, bis schließlich mit dem erneuten Aufsetzen des linken hinteren Hufs an der Stelle 34 nach ca. 1,2 sec bzw. nach Ablauf von 100% der Zyklusdauer 29 der nächste Zyklus beginnt (=0% der nächsten Zyklusdauer).

Bei der Analyse einer Vielzahl von Schrittfolgen hat sich herausgestellt, daß bei nicht lahmenden Reittieren die Zeitspanne zwischen dem Auftreten oder Abheben links hinten und rechts hinten immer ziemlich genau 50% der Zyklusdauer 29 beträgt. Gleiches gilt für die Zeitspanne zwischen dem Auftreten der Hufe der beiden vorderen Extremitäten. Dieses enge Sollmaß der Auftreffzeitpunkte um 50 % herum der paarigen Extremitäten wird erfindungsgemäß dazu benutzt, um eine Aussage treffen zu können, ob ein Reittier lahmt oder nicht.

Zur Erleichterung einer exakten Diagnose werden aus den Amplituden der erhaltenen elektrischen Signale erfindungsgemäß Größen abgeleitet, die für Zeitpunkte und/oder Zeitdauern charakteristisch sind, die mit den Bodenkontaktphasen in Beziehung stehen. Die von der linken und rechten Extremität des vorderen und/oder hinteren Extremitätenpaars stammenden Kenngrößen können dann miteinander verglichen werden, und die ermittelten Abweichungen lassen sehr genaue Aussagen über das Vorhandensein oder Fehlen einer Lahmheit zu. Als Kenngrößen eignen sich vor allem die Auftreffzeitpunkte der Hufe auf dem Erdboden, die Abhebezeitpunkte der Hufe vom Erdboden und die Stützdauern der Hufe, d.h. die Zeitabschnitte zwischen den Auftreff- und den Abhebezeitpunkten.

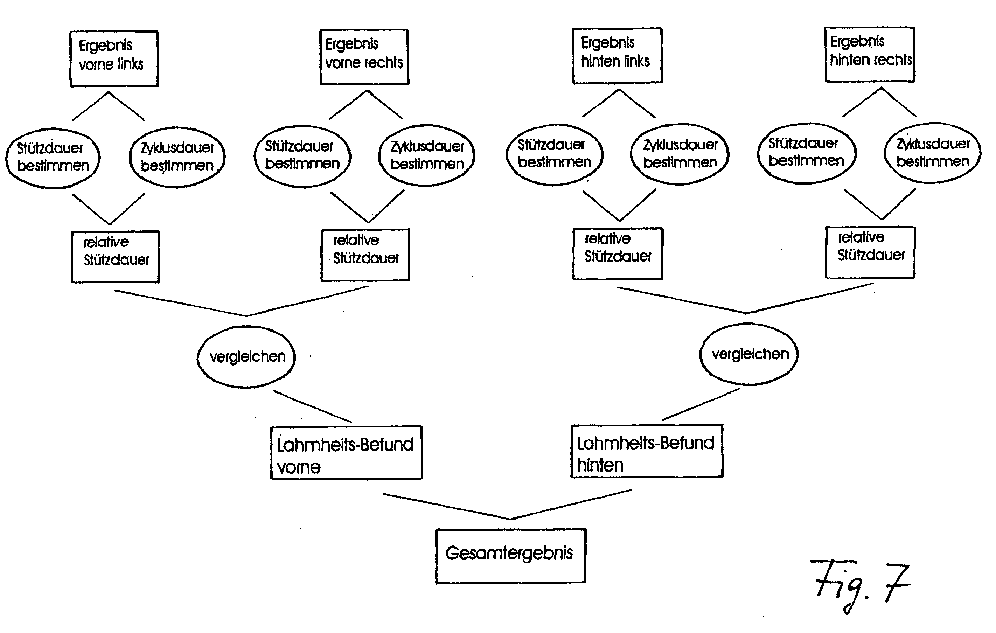

Bei den verwendeten Drucksensoren ergeben sich für jeden Huf und damit für jeden Kanal Meßsignale, die nach einer entsprechenden Glättung schematisch etwa denen entsprechen, die in Fig. 6 auszugsweise dargestellt sind. Eine Kenngröße in Form eines Auftreffzeitpunkts markiert hierbei bei entsprechender Polung des Sensors 4 jeweils einen Beginn 35 eines besonders steilen Anstiegs im Anschluß an eine Phase 36 längerer Konstanz im Signal, wobei die Phase 36 als Schwungphase bezeichnet wird, während welcher das Reittier den entsprechenden Huf in der Luft nach vorn bewegt. Der steile Anstieg im Drucksignal wird durch den Moment des Auftretens des Reittiers 1 mit dem entsprechenden Huf am Erdboden erzeugt, da der Sensor 4 in diesem Moment im Anschluß an eine Schwungphase 36 plötzlich stark komprimiert wird. Danach kommt eine der Stützdauer entsprechende Phase 37, während der der entsprechende Huf am Erdboden abgestützt ist. Der Beginn 38 eines steilen Abfalls im Drucksignal entspricht dagegen dem Abheben des Hufes vom Erdboden. Diese ebenfalls mit den Bodenkontaktphasen der Hufe des Reittiers in Beziehung stehende Kenngröße wird bei der nachfolgend geschilderten, einfachen Verarbeitung der Daten nicht ausgewertet.

Mit Bezug auf Fig. 6 wird nachfolgend ein Erkennungsverfahren geschildert, das dazu dient, den Zeitpunkt des Beginns 35 eines steilen Anstiegs in den Signalen zu erkennen. Hierzu werden die Signale unter Bildung von Differenzenquotienten nach der Zeit abgeleitet, und diejenigen Zeitpunkte werden als Beginn 35 des steilen Anstiegs im Signal verwendet, die für markante Sprünge einer lokalen Steigung von etwa Null auf einen betragsmäßig relativ hohen Wert charakteristisch sind, was im folgenden näher erläutert wird.

Der Differenzenquotient ist jeweils

$$f\,(t_{i+d}) - f\,(t_i)\,/\,(t_{i+d} - t_i),$$

wobei $f(t_{i+d})$ und $f(t_i)$ zwei Meß- bzw. Amplitudenwerte im diskretisiert vorliegenden Signal und $t_{i+d}$ und $t_i$ die zugehörigen Zeitpunkte sind, die z.B. eine Rasterweite $\underline{d}$ von 20 msec oder vier Meßwerten voneinander entfernt sind. Im Beispiel besteht das digitale Signal pro Kanal bei Anwendung einer Meßfrequenz von 200 Hz aus 9600 diskreten Werten $f(t_i)$, $f(t_{i+1})$ für i=1 bis 9600 (40 Schrittfolgen von je 1,2 Sekunden Dauer und 200 Werten pro Sekunde), wobei diese Werte einen zeitlichen Abstand von 5 msec besitzen (Meßfrequenz 200 Hz). Damit liegen jeweils drei Meßwerte innerhalb der Rasterweite $\underline{d}$, die bei der Quotientenberechnung übersprungen werden.

Das Signal wird sukzessiv mit der konstanten Rasterweite $\underline{d}$ der Zeitdauer ($t_{i+d}$ minus $t_i$) "durchkämmt", beginnend mit einer ersten Quotientenberechnung zwischen dem ersten vorliegenden Meßwert $f\,(t_{i=1})$ und einem zweiten Meßwert $f\,(t_{i+d=5})$, wobei $\underline{d}$ so gewählt ist, daß der durch den Wert des Quotienten repräsentierte Wert der lokalen Steigung im Signal einen Rückschluß auf das Auftreffverhalten des betroffenen Hufes ermöglicht. Bei zu gering gewählter Rasterweite $\underline{d}$ könnte das Ergebnis auch einen zufälligen Anstieg im Rauschen des Signals wiedergeben, während bei zu groß gewählter Rasterweite $\underline{d}$ der Zeitpunkt des Beginns 35 des Anstiegs möglicherweise nicht genau genug erfaßt wird. Ein Richtwert von etwa einem Prozent der Zyklusdauer 29, d.h. für das Beispiel 20 msec, sollte zumindest mit einer für die Gesamtauswertung der Signale hinreichenden Genauigkeit geeignet sein, feststellen zu können, wann das Reittier 1 jeweils mit seinen Hufen im Verlaufe seiner Fußungen auf den Erdboden auftritt.

Die Differenzquotienten werden also in hinreichender zeitlicher Auflösung gebildet. Anschließend werden sie jeweils paarweise aufeinanderfolgend verglichen. Ergibt sich dann ein erster Sprung des Quotienten von etwa Null bzw. einem vergleichsweise kleinen Wert auf einen vorzugebenden relativ hohen Wert oder höher, wobei dieser von der Aussteuerung des Signals abhängt, und bleiben gleichzeitig die Amplitudenwerte bei den nachfolgenden Zeitpunkten $t_i$ innerhalb einer gewissen Zeitspanne von etwa 5 % der Zyklusdauer 29 wenigstens gleich groß oder wachsen sie noch weiter, so wird derjenige Zeitpunkt gespeichert, der dem ersten hohen Steigungswert entspricht. Im Arbeitsspeicher 25 wird dieser Zeitpunkt somit als erster Zeitpunkt des Auftritts des Hufes am Erdboden, ausgedrückt als Zeitdifferenz zum gemeinsamen zeitlichen Nullpunkt, oder als erste Kenngröße abgespeichert.

Anschließend wird dasselbe Signal im Sinne wachsender Werte von i auf die gleiche Weise weiter durchsucht, wobei das oben beschriebene Erkennungsverfahren vorzugsweise für eine vorbestimmte Anzahl von Meßwerten unwirksam bleibt, um sicher zu sein, daß nicht eine zweite Kenngröße als Teil einer bereits überprüften Bodenkontaktphase fehlerhaft noch einmal erkannt wird. Diese Anzahl von Meßwerten könnte beispielsweise so groß sein, wie es 60 % der Meßwerte einer Schrittfolge entspricht (im Beispiel also 200 x 1,2 x 60% = 144).

Alternativ zur Unterdrückung des Signals für eine vorgewählte Anzahl von Meßwerten könnte auch das Auftreten eines steilen, längeren Abfalls 38 oder je nach Sensor ggf. das Auftreten eines Nulldurchgangs im Signal oder eine Schwungphase 36, d.h. eine längere Konstanz des Signals auf einem Wert um Null herum als Kriterium herangezogen werden, das erfüllt sein muß, bevor das Erkennungsverfahren zum Zwecke der Feststellung des Auftrittszeitpunkts der nachfolgenden Bodenkontaktphase wieder wirksam geschaltet wird. Es sei in diesem Zusammenhang auf die dem Fachmann geläufigen Methoden der digitalen Signalverarbeitung zur Erkennung bestimmter Signalverläufe aus einem Meßsignal verwiesen.

Anschließend wird der Zeitpunkt der nachfolgenden Kenngröße analog zur obigen Verfahrensweise bestimmt und im Arbeitsspeicher 25 gespeichert. Auf diese Weise wird bis zum Ende des Signals fortgefahren, wodurch so viele Kenngrößen ermittelt werden, wie Auftrittzeitpunkte des zugehörigen Hufs vorliegen.

Um die Genauigkeit der Ermittlung dieser Zeitpunkte zu erhöhen, wird das beschriebene Verfahren noch einmal mit der Maßgabe durchgeführt, daß Meßwerte $f(t_i)$ zur Berechnung der Differenzenquotienten verwendet werden, die zu den Zeitpunkten $t_{i+1/2d}$ anstatt zu den Zeitpunkten $t_i$ gehören, d.h. um die Hälfte der Rasterweite $\underline{d}$ später liegen. Die Ergebnisse des zweiten Durchgangs werden mit denen des ersten Durchgangs jeweils paarweise gemittelt und im Arbeitsspeicher 25 abgespeichert. Somit sind die Zeitpunkte des Beginns steiler Anstiege für einen Kanal hinreichend genau ermittelt.

Die Signale der restlichen Kanäle werden in gleicher Weise unter Anwendung der oben beschriebenen Auswertung nach steilen Anstiegen durchsucht, und die ermittelten Zeitpunkte werden so im Arbeitsspeicher 25 gespeichert, daß, bezogen auf den festen zeitlichen Nullpunkt, alle Zeitpunkte gespeichert sind, zu denen Hufe des Reittiers 1 am Erdboden auftreffen.

Durch die Bildung der Differenzen zwischen jeweils aufeinanderfolgenden Zeitpunkten eines willkürlich gewählten Kanals können nun die Zyklusdauern 29 als weitere Kenngrößen berechnet und im Arbeitsspeicher 25 nach ihrer zeitlichen Abfolge abgespeichert werden, wobei angenommen wird, daß sich von Kanal zu Kanal keine wesentlichen Unterschiede in den Zyklusdauern 29 ergeben, d.h. unbedeutend ist, ob die Zyklusdauer 29 anhand der Auftreffzeitpunkte z.B. des linken Hinterhufs oder des rechten Vorderhufs ermittelt wird. Zur Kontrolle der so ermittelten Zyklusdauern könnten zusätzlich die Zyklusdauern von den verbleibenden Kanälen berücksichtigt und die einzelnen Zyklusdauern beispielsweise durch Mittelung über die Zyklusdauern aus den anderen Kanälen berechnet werden.

Erfindungsgemäß wird nun beispielsweise weiter vorgesehen, die quasi-kontinuierliche Bewegung des Reittiers 1 in einzelne Schrittfolgen zu zergliedern, damit es möglich ist, diese einzeln und unabhängig voneinander zu analysieren. Dafür werden die Auftreffzeitpunkte kanalweise nach ihrer zeitlichen Abfolge geordnet und jeweils nur noch auf ihr Erscheinen in der zugehörigen Schrittfolge bezogen, indem untersucht wird, mit welchem zeitlichen Abstand vom Beginn des zugehörigen Zyklus die Kenngröße auftritt. Diese Zeitspanne wird dann mit der zugehörigen Zyklusdauer 29 in Beziehung gesetzt und z.B. in einen Prozentsatz der Zyklusdauer 29 umgerechnet. Dabei könnte beispielsweise für die siebte Schrittfolge festgestellt werden, daß der Auftreffzeitpunkt Nr.7 des linken Vorderhufs nach Ablauf von 20% der zugehörigen Zyklusdauer 29 oder der Auftreffzeitpunkt Nr.7 des rechten Vorderhufs nach Ablauf von 85% der zugehörigen Zyklusdauer 29 (Nr.7) auftrat. Diese Werte sind somit nicht mehr auf den oben genannten gemeinsamen Nullpunkt, sondern auf den Beginn des jeweils zugehörigen Schrittfolgezyklus bezogen.

Anschließend werden die für alle z.B. vierzig Schrittfolgen erhaltenen Prozentwerte eines Kanals von den entsprechenden Prozentwerten eines anderen Kanals abgezogen, beim obigen Beispiel also z.B. die Prozentwerte des Kanals des rechten Vorderhufs von denen des Kanals für den linken Vorderhuf (beim obigen Beispiel 85% - 20% = 65 %). Die erhaltenen Kenngrößen in Form von Differenzen werden gespeichert. Dabei ergibt sich, daß im obigen Beispiel ein Differenzwert von 65 % anstatt des anhand der Fig. 5 erläuterten, für ein gesundes Reittier normalen Prozentwerts von 50% erhalten wird.

Schließlich werden vierzig Einzelergebnisse -eines für jede der gemessenen Schrittfolgen- ausgegeben, wodurch beispielsweise eine Reihe von Prozentzahlen auf dem Display 26 der Station 14 erscheint, wobei ein Ausschnitt davon wie folgt aussehen könnte:

63% 62,5% 65% 66% 62% 64,7% 68% 63% 64,7% 65% .

Der Anwender des erfindungsgemäßen Verfahrens, der die Station 14 z.B. in Form eines Handterminals in der Hand hält und das Reittier 1 während der Messung beobachtet, kann somit aus dieser Zahlenreihe sofort ersehen, daß alle Kenngrößen größer als 50% sind. Er erkennt unmittelbar (vgl. Fig. 5), daß es immer länger als eine halbe Zyklusdauer 29 von 50% dauert, bis nach Auftreffen des linken Vorderhufes am Erdboden auch der rechte Huf auftrifft. Wenn daher bei Reittieren, die nicht lahmen, zwischen den zugehörigen Auftreffzeitpunkten zweier Hufe des gleichen Extremitätenpaares (z.B. des vorderen) immer etwa die Hälfte einer Zyklusdauer 29 liegt, wobei Einzelabweichungen bis 5% noch als "gesund" betrachtet werden, dann kann aus den stets oberhalb von 60 % liegenden Werten unmittelbar darauf geschlossen werden, daß das Reittier 1 den rechten Huf immer zu spät aufsetzt und es diesen dadurch zu Lasten des anderen schont. Aus dem Ausmaß der Abweichung läßt sich folgern, daß das Reittier 1 lahmt. Da eine mittlere Abweichung von etwa 3% von der 50 %-Marke noch als gesund betrachtet werden kann, handelt es sich im vorliegenden Fall bereits um höhergradiges Lahmen.

Zusätzlich zu den Einzelwerten kann auch das Mittel über die obige Zahlenreihe errechnet und z.B. ein Durchschnittswert von 64,4% auf dem Display 26 ausgegeben werden. Dadurch werden zufällig entstandene Abweichungen herausgemittelt, und der Anwender kann eine systematische Abweichung vom Normal- oder Idealwert von 50 % sofort erkennen.

Derselbe Vergleich, der für die beiden Vorderextremitäten oben erstellt wurde, kann unter Verwendung derselben Meßdatenbasis auch für die beiden hinteren Extremitäten erstellt werden. Als Kenngrößen können alternativ auch z.B. die unter Verwendung des Beginns 38 des steilen Abfalls (Fig. 6) ermittelten Abhebezeitpunkte verwendet werden, deren Berechnung analog zur Berechnung der Auftreffzeitpunkte erfolgen kann.

Eine vorteilhafte Variante bzw. Weiterbildung des erfindungsgemäßen Verfahrens wird im folgenden anhand eines weiteren Meßbeispiels erläutert, bei dem als Kenngrößen die Stützdauern, die sich aus den Differenzen der Zeitpunkte des Auftreffens und der Zeitpunkte des nachfolgenden Abhebens desselben Hufes vom Erdboden ergeben, verwendet und paarweise für die Vorderextremitäten oder die Hinterextremitäten miteinander verglichen werden. Es werden hier somit Kenngrößen verwendet, die für Zeitdauern anstatt für Zeitpunkte der Bodenkontaktphasen charakeristisch sind, d.h. maßgeblich für eine Beurteilung der Lahmheit eines Reittiers 1 ist hier nicht der Beginn oder das Ende des Auffussens, sondern die jeweilige rechte und linke Stützdauer innerhalb der gleichen Schrittfolge. Die zusätzliche Ermittlung der Abhebezeitpunkte geschieht dabei in Analogie zum Auffinden des Beginns 35 des steilen Anstiegs z.B. mit der Maßgabe, daß Sprünge von negativen Werten der lokalen Steigung auf Werte um Null vorliegen und die Amplitudenwerte vorher in einem gewissen Bereich von z.B. etwa 5% der Zyklusdauer 29 auf einem relativ hohen Wert (Phase 37) gelegen haben müssen.

Die Stützdauern werden vorzugsweise mit der zugehörigen Zyklusdauer 29 normiert, dh. in Prozentwerte der jeweiligen Zyklusdauern umgerechnet. Diese Kenngrößen werden dann z.B. dadurch, daß die für die rechten bzw. linken Hufe erhaltenen Stützdauern voneinander abgezogen werden, miteinander verglichen. Ein möglicher Auszug aus einer ausgegebenen Zahlenreihe wäre der folgende:

- 5,3% -4,7% -6,2% -3,8% -6,0% -3,1% -5,9% -5,9% -7,8% -6,3% .

Die Ausgabe kann wie oben als Einzel- und/oder Durchschnittsergebnis erfolgen.

Weichen die erhaltenen Differenzen (z.B. links vorn minus rechts vorn) im Einzelfall oder im Durchschnitt um einen gewissen Prozentsatz von einem als ideal angesehenen Wert - nämlich Null - ab, so kann daraus geschlossen werden, daß das eine Bein des Paars jeweils um diesen Prozentsatz kürzer oder länger als das andere Bein desselben Paars belastet wurde. Daraus kann wiederum auf eine beim Reittier 1 vorhandene Lahmheit geschlossen werden. Im vorliegenden Beispiel ist die Stützdauer links vorn jeweils kürzer als die rechts vorn, d.h. das Reittier 1 schont seine linke Extremität zu Lasten der rechten.

Die Vergleiche der zu den rechten und linken Extremitäten gehörigen Kenngrößen können manuell oder auch automatisch durchgeführt werden. Nach einer besonders bevorzugten Ausführungsform ist jedoch die beschriebene Auswerteeinheit 18 bzw. ihr Mikrocomputer softwaremäßig so eingerichtet, daß die beschriebenen Verfahrensschritte automatisch ablaufen und daß vom Display 26, dem Drucker 27 oder dgl. sofort die oben erläuterten Prozentwerte ausgegeben werden. Ein bevorzugter Ablaufplan für ein derartiges Programm ist grob schematisch in Fig. 7 dargestellt.

Die beiden oben geschilderten Verfahren liefern eine zeitliche Beschreibung bestimmter Teile der Bewegung der Hufe des Reittiers 1 mit bestimmten Zeitpunkten der Bodenkontaktphasen der Hufe als Basisgrößen. Die Verfahren können auf der Basis der gesammelten Daten unter Einbeziehung der absoluten, also nicht auf die Zyklusdauer bezogenen Stützdauern in das Endergebnis verfeinert werden. Sie sind unter Verwendung einer ausreichenden Meßfrequenz zur Auflösung der Meßkurven, insbesondere der markanten Stellen des Beginns oder des Endes steiler Anstiege oder Abfälle auch für andere Gangarten des Reittiers 1 wie z.B. Trab brauchbar, wodurch die Abhängigkeit des Grades der Lahmheit vom Bewegungstempo und der Gangart erkannt werden kann. Daraus folgt, daß der Begriff "Schrittfolge" nicht einschränkend auf die Gangart "Schritt" bei Pferden beschränkt ist.

Unter Einbeziehung weiterer statistischer Größen wie der Standardabweichung, der Medianwerte etc. und unter Einbeziehung üblicher statistischer und stochastischer Auswertemethoden (Regressionsanalyse, Quartilbildung, Verteilungsfunktionen, etc.) können die Verfahren weiter verfeinert werden, um genauere Aussagen über den Grad der Lahmheit, die Regelmäßigkeit oder Unregelmäßigkeit der festgestellten Lahmheiten oder über die Verläßlichkeit der Messung und, bei wiederholter Messung im zeitlich längeren Abstand, über den Verlauf einer Heilung der betroffenen lahmenden Extremität etc., machen zu können. Dabei versteht sich, daß zusätzlich auch die von gleichseitigen oder von allen Extremitäten erhaltenen Kenngrößen in ihrem zeitlichen Ablauf miteinander verglichen werden können. Dabei ist beachtlich, daß die Zeitspanne zwischen dem Auftreten der gleichseitigen Extremitäten, d.h. links hinten und links vorn bzw. rechts hinten und rechts vorn, nicht zwangsläufig bei 25% bzw. 75 % der Zyklusdauer 29 liegen müssen. Wie das Beispiel der Fig. 5 zeigt, ergeben sich z.B. Werte von etwa 19 % und 69 %, die je nach Rasse und individuellen Merkmalen des Reittieres schwanken können.

Der entscheidende Vorteil zu anderen Verfahren mit gleicher Sensibilität bei der Lahmheitsbeurteilung bei Reittieren ist die Möglichkeit, die gesammelten Daten auf sehr einfache Weise auswerten zu können, da die Meßsignale eine einfache, periodische Struktur besitzen und die Amplituden der Signale nicht absolut oder unter Verwendung von Schwellwerten, sondern nur zur Feststellung des Beginns oder des Endes steiler Anstiege oder Abfälle im Signal ausgewertet werden müssen. Daher sind die Signale auf einfache Weise eindeutig interpretierbar. Infolgedessen werden keine extrem aufwendigen Programme benötigt, um die Daten auswerten zu können.

Ebenso vorteilhaft ist dabei, daß das Reittier 1 nur durch ein Mindestmaß an Verkabelung und Meßeinrichtungen an seinem Körper belastet wird und daher kaum etwas von der Messung merkt.

Das oben beschriebene Auswertungsverfahren beruht auf der Erzeugung von elektrischen Signalen gemäß Fig. 6, die bei Anwendung von Drucksensoren 4 bzw. Dehnungsmeßstreifen 11 in Fig. 1 und 2 erhalten werden. Vorzugsweise werden an den Seitenwänden der Hufe angebrachte Dehnungsmeßstreifen (11 in Fig. 1 und 2) angewendet, die die Dehnungen bzw. Stauchungen der Hufwand messen und daher unabhängig davon sind, ob der Huf auf einen weichen oder harten Boden tritt, ob er mit einem Eisen beschlagen ist oder ob sonstige das Meßsignal beeiflussende Merkmale vorhanden sind, wie es bei Anwendung der in Fig. 2 gezeigten Drucksensoren 4 der Fall sein könnte.

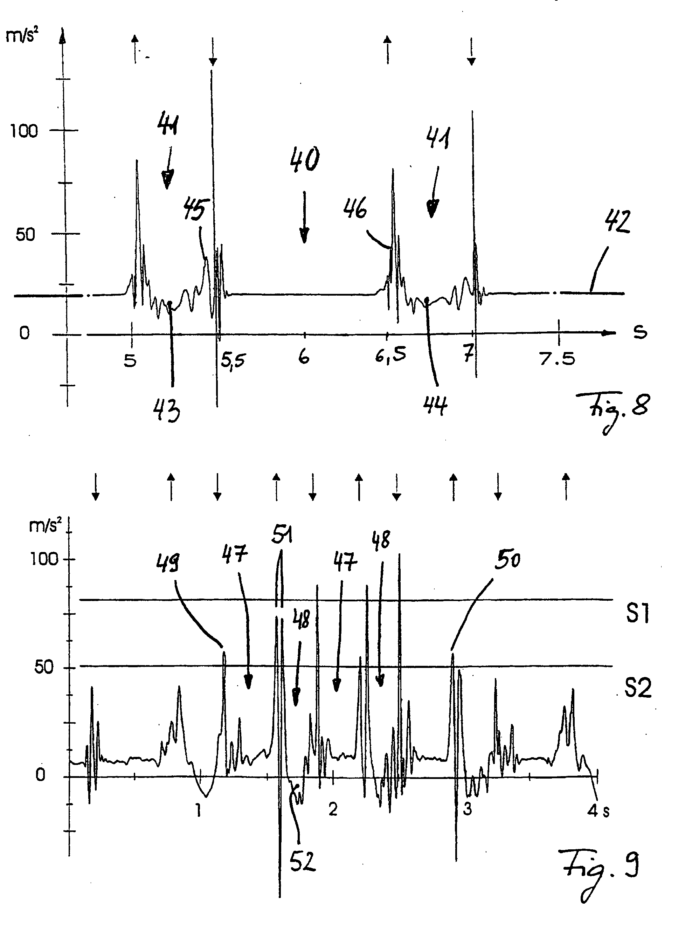

Alternativ ist es aber auch möglich, an der Außenseite des Hufs oder am Röhrbein jeweils einen Beschleunigungssensor (vgl. auch US-PS 4 774 679) anzubringen, der die beim Auf- bzw. Abfußen auftretenden Beschleunigungswerte in $m/s^2$ mißt. Als Beschleunigungssensoren eignen sich z.B. solche, die unter der Bezeichnung B 12/500 von der Fa. Hottinger Baldwin Meßtechnik, Darmstadt, vertrieben werden. Bei Anwendung derartiger Beschleunigungssensoren werden kontinuierliche elektrische Signale erhalten, wie sie in Fig. 8 und 9 dargestellt sind. Charakteristisch für diese Signale ist der Umstand, daß sie zahlreiche Minima und Maxima aufweisen, die die Auswertung erschweren und nicht, wie in US-PS 4 774 679 irrtümlich angegeben ist, einfach durch die Wahl eines Schwellwerts zu brauchbaren, für Zeitpunkte oder Zeitdauern der Bodenkontaktphasen chrakteristischen Kenngrößen führen.

Fig. 8 zeigt den Ausschnitt eines elektrischen Signals, das bei der Gangart Schritt eines Pferdes und bei Anbringung eines Beschleunigungssensors an einem Huf eines Pferdes erhalten wurde, wobei längs der Abzisse die Zeit in Sekunden und längs der Ordinate die Beschleunigung in $m/s^2$ aufgetragen ist. Die oberhalb der Kurve dargestellten Pfeile bedeuten eine Auftreffen des zugehörigen Hufs auf dem Erdboden, wenn sie nach unten gerichtet ist, bzw. ein Abheben des zugehörigen Hufs vom Erdboden, wenn sie nach oben gerichtet sind.

Nach Fig. 8 hat die Kurve etwa bei den Zeitpunkten 5, 5,5, 6,5 und 7 jeweils wenigstens ein deutlich ausgeprägtes Maximum, wobei die Zeitpunkte 5 und 6,5 dem Beginn eines Abhebens und die Zeitpunkte 5,5 und 7 dem Beginn eines Auftreffens zugeordnet sind. Zwischen den Zeitpunkten 5,5 und 6,5 liegt somit eine Stützphase 40, während zwischen den Zeitpunkten 5 und 5,5 bzw. 6,5 und 7 jeweils eine Schwungphase 41 liegt. Aus dem Meßsignal ist außerdem erkennbar, daß zwar für jedes Auf- und Abfußen ein deutliches Maximum erhalten wird, d.h. die Auf- und Abfußsignale in dieselbe Richtung gehen, und daß die Auffußmaxima in der Regel größer als die Abfußmaxima sind. Da jedoch im dargestellten Signal die Beschleunigungswerte über die Zeitachse hinweg stark schwanken, ist es nicht einfach möglich, die Auftreff- bzw. Abhebezeitpunkte durch die Wahl eines Schwellwertes zu ermitteln. Eine brauchbare Auswertung kann jedoch auf die folgende Weise vorgenommen werden.

Es wird zunächst von der Erkenntnis ausgegangen, daß das Signal nach Fig. 8 um eine Linie 42 schwankt, die oberhalb der x-Achse liegt und einem Wert entspricht, der sich aus einem bei Beschleunigungssensoren beachtlichen Wert für die Erdbeschleunigung ergibt. Die Linie 42 wird daher nachfolgend als Bezugslinie verwendet. Außerdem ist aus Fig. 8 erkennbar, daß das Meßsignal während der Schwungphasen 41 vergleichsweise stark um die Linie 42 schwankt, während der Stützphasen 40 dagegen praktisch denselben Wert wie die Linie 42 annimmt. Dieser Umstand wird erfindungsgemäß für die Auswertung ausgenutzt.

Das Signal wird zunächst geglättet, um die zahlreichen kleinen Unebenheiten herauszufiltern. Sodann wird das Gesamtsignal untersucht und z.B. darauf überprüft, wieviele bzw. wie große Maxima auch nach der Glättung noch vorhanden sind. Anhand dieser Überprüfung wird dann z.B. entschieden, daß der eigentlichen Auswertung nur z.B.

solche Maxima zugrunde gelegt werden, deren Amplitudenwerte wenigstens 10 % der maximal vorkommenden Beschleunigung betragen. Dabei sollte die Auswahl dieser Maxima anhand der individuell vorliegenden Meßreihe so gewählt werden, daß keine für die Auftreff- bzw. Abhebezeitpunkte wesentlichen Maxima unterdrückt werden. Die Ermittlung des Beginns der Maxima kann auf die oben beschriebene Weise durch die Bildung von Differenzenquotienten erfolgen. Entsprechend können die verbleibenden Maxima wie beschrieben mit den zugeordneten Zeiten gespeichert werden.

Das Signal nach Fig. 8 wird nun auf die Lage der Schwungphasen 40 untersucht. Hierzu wird vorausgesetzt, daß eine Schwungphase dann vorliegt, wenn erstens ein Minimum erkannt wird und zweitens die Amplitude des Meßsignals im Bereich dieses Minimums für eine Zeitdauer von wenigstens 0,1 Sekunden negativ, d.h. kleiner ist, als dem Wert der Linie 42 entspricht. In Fig. 8 trifft dies z.B. für zwei Bereiche 43 und 44 zu. Ist eine Schwungphase 41 erkannt, wird das nächste oberhalb der genannten Schwelle liegende Maximum (hier z.B. ein Maximum 45) als ein Auffussen, d.h. als ein Wert interpretiert, der in der obigen Beschreibung etwa dem Beginn 35 des Auffussens (Fig. 6) entspricht. In ähnlicher Weise wird das Vorhandensein einer Stützphase 40 dadurch definiert, daß das Meßsignal für eine Zeitdauer von z.B. 0,2 Sekunden im wesentlichen dem Wert der Linie 42 entspricht. Ist eine Stützphase 40 ermittelt, wird das erste oberhalb der Schwelle liegende Maximum (hier z.B. ein Maximum 46) nach dieser Stützdauer 40 als Abhebezeitpunkt erkannt (analog dem Beginn 38 des Abfussens in Fig. 6). Zweckmäßig wird für jeden Auf- und Abfußzeitpunkt bzw. jede Scherung- und Stützphase auf dieselbe Weise ausgewertet.

Sind auf diese Weise die Auftreff- und/oder Abhebezeitpunkte ermittelt, kann zur Diagnose der Lahmheit auf dieselbe Weise vorgangen werden, wie oben in Verbindung mit Fig. 6 beschrieben wurde.

Die Anbringung eines Beschleunigungssensors am Huf eines Reittiers bringt den aus Fig. 8 ersichtlichen Vorteil mit sich, daß sich die Stützphasen 40 und Schwungphasen 41 optisch relativ gut voneinander unterscheiden lassen, so daß eine brauchbare Auswertung im Prinzip schon dadurch möglich ist, daß die Meßkurven mit einem Schreiber oder dgl. auseinander gezogen und manuell ausgewertet werden. Ist der Beschleunigungssensor dagegen am Röhrbein angeordnet, dann kommen zu den durch das Auf- und Abfußen verursachten Schwingungen bzw. Beschleunigungen noch diejenigen Schwingungen hinzu, die durch die zwischen den Hufen und Röhrbeinen befindlichen, federnden Fesselgelenke von Reittieren verursacht werden. Das ist aus Fig. 9 ersichtlich, wonach zwischen wiederum durch Pfeile gekennzeichneten Stützund Schwungphasen 47 bzw. 48 zahlreichen Maxima und Minima liegen, die die Aufund Abfußzeitpunkte oder andere charakteristische Kenngrößen nicht ohne weiteres erkennen lassen. Insbesondere wäre die bloße Einführung von Schwellwerten, wie sie z.B. durch Linien S1 und S2 gekennzeichnet sind, wenig hilfreich, da je nach Fall z.B. auch Maximn 40,50 unterdrückt werden (Schwellwert S1), die für das Auf- oder Abfussen charakteristisch sind, oder Doppel- und Mehrfachmaxima 51 stehen bleiben (Schwellwert S1), die nicht vorhandene Auf- und Abfußzeitpunkte vortäuschen würden.

Demgegenüber wird erfindungsgemäß vorgeschlagen, die Schwungphasen 48 z.B. dadurch zu ermitteln, daß charakteristische Minima 52 (Fig. 9) herangezogen werden und gefordert wird, daß eine Schwungphase dann vorliegt, wenn die Amplitude des Meßsignals im Bereich dieses Minimums 51 für eine vorgewählte Zeitlang kleiner als ein vorgewählter Wert ist. Die Stützphasen 48 könnten dagegen durch die Forderung erkannt werden, daß z.B. für mehr als 0,2 Sekunden kein oberhalb einer vorgewählten Beschleunigung liegendes Maximum vorliegt und gleichzeitig keine Schwungphase 47 erkannt wird. Auch durch derartige Auswertungen können somit Kenngrößen erhalten werden, die für den Bodenkontaktphasen zugeordnete Zeitpunkte und/oder Zeitdauern charakteristisch sind.

Eine für die Zwecke der Erfindung in Verbindung mit Meßsignalen nach Fig. 9 brauchbare Software wird z. B. unter der Bezeichnung "DIA/DAGO-PC" von der Fa. Gesellschaft für Strukturanalyse mbH, Aachen, Deutschland, hergestellt und vertrieben. Eine derartige, auf üblichen Personalcomputern anwendbare Software ermöglicht die Ermittlung der Maxima und Minima mit und ohne Vorgabe von Schwellwerten unter gleichzeitiger Zuordnung der jeweiligen Zeitpunkte.

Die Erfindung ist nicht auf die beschriebenen Auführungsbeispiele beschränkt, die auf vielfache Weise abgewandelt werden können.

Beispielsweise könnte die Funktionseinheit 7 außer oder anstelle der Analog/Digital-Wandlung der Meßsignale auch andere Aufgaben erfüllen, insbesondere im Hinblick auf die weitere Bearbeitung und Auswertung der Signale. Weiter könnte vorgesehen sein, daß die Station 14 ab einer Durchschnittsabweichung von z.B. mehr als 6% ein zusätzliches optisches oder ein akustisches Signal abgibt. Weiter können bei guter Glattheit der Meßsignale die Zeitpunkte eines Beginns 35 eines steilen Anstiegs oder des Beginns 38 eines steilen Abfalls (oder jeweils der Enden davon) auch aufgrund eines Vergleichs von aufeinanderfolgenden Funktionswerten ermittelt werden, ohne daß gnale auch als Analogsignale für jeden Huf kanalweise getrennt gesendet, in der Station 14 analog gefiltert und erst dann digitalisiert und synchronisiert werden. Für den Fall, daß keine Sendeeinheiten verfügbar sind, die einerseits ausreichend klein und für die Unterbringung am Röhrbein geeignet sind, andererseits aber auch über eine ausreichend große Reichweite verfügen, um die Signale bis zur Station 14 zu übertragen, besteht eine bevorzugte Variante der Erfindung darin, eine leistungsstarke, zentrale Sendeeinheit am Rücken des Reittiers, am Reiter selbst, am Sattel od. dgl. anzubringen und dieser eine Empfangseinheit vorzuschalten, die die von kleinen, an den Beinen des Reittiers angebrachten Sendeeinheiten gesendeten Signale aufnimmt und an die zentrale Sendeeinheit weitergibt. Möglich wäre es natürlich auch, die Meßsignale zunächst in einem am Reittier montierten Speicher zu sammeln und später in einer separaten Station 14 auszuwerten.

Weiter ist das Verfahren auch in der Weise veränderbar, daß als Kenngrößen die Zeitpunkte der Mitten der Stützdauern oder analog zu Fig. 8 und 9 beliebige andere, markante Signalstellen anstelle oder zusätzlich zu den Zeitpunkten des Beginns des steilen Anstiegs oder des Beginns des steilen Abfalls ausgewertet werden, um z.B. das Überschwingen der Sensoren 4 zu kompensieren. Im übrigen hängt die Frage, welche spezielle Kenngröße für die Beurteilung der Gangeigenschaften am besten kennzeichnend ist, weitgehend auch vom Einzelfall ab und kann je nach Diagnoseziel auch unterschiedlich gewählt werden.

Die erfindungsgemäße Vorrichtung kann durch entsprechende Logiksteuerungen und Speichermittel auch dazu eingerichtet sein, die Daten aus mehreren Einzelmessungen zu speichern und die Daten dann in ihrer Gesamtheit auszuwerten. Dies kann insbesondere dann wichtig sein, wenn das Gelände, auf dem die Messungen stattfinden, zu klein ist, um etwa 40 brauchbare Schrittfolgesequenzen an einem Stück zuzulassen.

Anstelle der beispielsweise beschriebenen Sensoren können auch andere Sensoren, z.B. Kraftsensoren auf Basis einer Silicon-Micromembran der Fa. ISM, Freiburg/i.Br., verwendet werden, die zusätzlich eine Quantifizierung des Signals und nicht nur eine qualitative Auswertung im Sinne einer Unterscheidung zwischen Auftreten und Nicht-Auftreten zulassen. Sollen Messungen mit einer höheren Frequenz als ca. 200 Hz pro Kanal durchgeführt werden, um beispielsweise die Bewegungsanalyse des Reittiers 1 auf andere Gangarten, insbesondere die Gangart Trab, auszudehnen zu können, in denen die Stützdauern kürzer sind als im Schritt, so kann es sich empfehlen, beispielsweise piezokeramische Druckaufnehmer zu verwenden.

Weiter ist es möglich, die anfallenden Meßdaten in der Station 14 nur zu speichern und alle weiteren Auswertungen mit einem oder mehreren PC-Programmen zu einem späteren Zeitpunkt durchzuführen. Darüber hinaus ist es möglich, die von der Station 14 gesammelten und gespeicherten Werte gleichzeitig mit der Messung oder zeitlich versetzt über Funk oder Kabel an einen PC zu übertragen, auf dem für eine tiefer gehende Auswertung der Daten ein geeignetes Programm installiert ist. Diese und weitere Abwandlungen der Vorrichtung, insbesondere der hardwaremäßigen Ausgestaltung der Auswerteeinheit 18 sowie Verfeinerungen des Verfahrens oder einzelner ihrer Schritte oder Abwandlungen, die sich durch eine Kombination einzelner Merkmale von ihnen ergeben, sind von der Erfindung mitumfaßt. Außerdem versteht sich, daß die einzelnen Merkmale der Erfindung auch in anderen als den beschriebenen und in der Zeichnung dargestellten Kombinationen verwendet werden können.

**Patentansprüche**

1. Vorrichtung zur Erzeugung von für die Lahmheitsdiagnose bei einem Reittier (1) geeigneten Kenngrößen, die mit Bodenkontaktphasen der zu den Extremitäten gehörigen Hufe des Reittiers (1) während der Fußungen in Beziehung stehen, mit einer Sensoreinrichtung, die wenigstens zwei an zwei ausgewählten Extremitäten des Reittiers (1) anzubringende Sensoren (4,11) zur Erzeugung der elektrischen Signale enthält, und mit einer elektronischen Auswerteeinheit (18) zur Erzeugung der Kenngrößen aus diesen elektrischen Signalen, **dadurch gekennzeichnet, dass** die Auswerteeinheit (18) zur Erzeugung von solchen Kenngrößen aus den Amplitudenwerten der elektrischen Signale eingerichtet ist, die für den Bodenkontaktphasen zugeordnete Zeitpunkte und/oder Zeitdauern charakteristisch sind, und die Auswerteeinheit (18) Mittel zur Erzeugung von abgeleiteten Kenngrößen durch Vergleich derjenigen Kenngrößen aufweist, die von der linken und rechten Extremität des vorderen und/oder hinteren Extremitätenpaars stammen.

2. Vorrichtung nach Anspruch 1, **dadurch gekennzeichnet, dass** sie zur Ermittlung der zeitlichen Abweichungen der von unterschiedlichen Extremitäten eines Extremitätenpaars stammenden Kenngröße eine Zeitmess- und Synchronisiereinrichtung (22) enthält, die eine zeitliche Einordnung der einzelnen Kenngrößen während mehrerer Schrittfolgen ermöglicht.

3. Vorrichtung nach einem der Ansprüche 1 oder 2, **dadurch gekennzeichnet, dass** die Sensoreinrichtung mit deiner A/D-Wandlereinrichtung (7) gekoppelt ist.

4. Vorrichtung nach einem der Ansprüche 1 bis 3, **dadurch gekennzeichnet, dass** sie Sendemittel (12) zur drahtlosen Übertragung der von den Sensoren (4,11) abgegebenen Signale oder daraus abgeleiteter Signale vom Reittier (1) an eine die Auswerteeinheit (18) enthaltende Station (14) aufweist.

5. Vorrichtung nach Anspruch 4, **dadurch gekennzeichnet, dass** sie wenigstens einen mit der Station (14) gekoppelten Speicher (23,25) zum Speichern der empfangenen Signale und/oder Daten sowie zum Reproduzieren der Ergebnisse enthält.

## Claims

1. Device to generate, for diagnosis of lameness in the case of a riding animal (1), suitable characteristic quantities which are related to ground contact phases of the hooves, belonging to the extremities, of the riding animal (1) during the footfalls, with a sensor facility which includes at least two sensors (4, 11) to be attached to two selected extremities of the riding animal (1) to generate the electrical signals, and with an electronic analysis unit (18) to generate the characteristic quantities from these electrical signals, **characterized in that** the analysis unit (18) is set up to generate such characteristic quantities from the amplitude values of the electrical signals, which are characteristic of the instants and/or durations which are associated with the ground contact phases, and that the analysis unit (18) has means for generating derived characteristic quantities by comparing those characteristic quantities which originate in the left-hand and right-hand extremities of the front and/or rear extremity pair.

2. Device according to Claim 1, **characterized in that** to determine the temporal differences of the characteristic quantity which originates in different extremities of an extremity pair, it includes a time measurement and synchronisation device (22), which makes it possible to order the individual characteristic quantities in time during multiple step sequences.

3. Device according to one of Claims 1 or 2, **characterized in that** the sensor facility is connected to an A/D converter (7).

4. Device according to one of Claims 1 to 3, **characterized in that** it has transmission means (12) for wireless transmission of the signals which the sensors (4, 11) output or signals derived from them from the riding animal (1) to a station (14) which contains the analysis unit (18).

5. Device according to Claim 4, **characterized in that** it includes at least one memory (23, 25) which is connected to the station (14) to store the received signals and/or data and to reproduce the results.

## Revendications

1. Dispositif pour produire des paramètres appropriés pour le diagnostic de la boiterie d'un animal de selle (1), qui sont en relation avec des phases de contact au sol des sabots de l'animal de selle (1) appartenant aux extrémités pendant les foulées, comprenant un dispositif capteur qui comprend au moins deux capteurs (4, 11) destinés à être placés à deux extrémités choisies de l'animal de selle (1) pour produire les signaux électriques, et une unité d'analyse électronique (18) destinée à produire les paramètres sur la base de ces signaux électriques, **caractérisé en ce que** l'unité d'analyse (18) est construite pour produire de tels paramètres en se basant sur les amplitudes des signaux électriques qui sont caractéristiques des instants et/ou durées correspondant aux phases de contact au sol et l'unité d'analyse (18) comporte des moyens pour produire des paramètres dérivés issus de la comparaison des paramètres qui proviennent de l'extrémité gauche et de l'extrémité droite de la paire d'extrémités avant et/ou de la paire d'extrémités arrière.

2. Dispositif selon la revendication 1, **caractérisé en ce qu'**il comprend, pour obtenir les variations dans le temps des paramètres provenant d'extrémités différentes d'une paire d'extrémités, un dispositif de mesure du temps et de synchronisation (22) qui permet d'ordonner les paramètres dans le temps pendant plusieurs suites de pas.

3. Dispositif selon l'une des revendications 1 ou 2, **caractérisé en ce que** le dispositif capteur est couplé à un dispositif convertisseur A/N (7).

4. Dispositif selon l'une des revendications 1 à 3, **caractérisé en ce qu'**il comprend des moyens d'émission (12) pour la transmission sans fil des signaux issus des capteurs (4, 11), ou de signaux qui en sont dérivés, de l'animal de selle (1) à une station (14) qui comprend l'unité d'analyse.(18).

5. Dispositif selon la revendication 4, **caractérisé en ce qu'**il comprend au moins une mémoire (23, 25) couplée à la station (14) et destinée à mémoriser les signaux reçus et/ou les données ainsi qu'à reproduire les résultats.

Fig. 1

Fig. 2

Fig. 3

Fig. 4

Fig. 5

Fig. 6

Fig. 7

Fig. 8

Fig. 9